# EUROPEAN PATENT APPLICATION

(11) **EP 1 832 219 A1**
(43) Date of publication of application: **12.09.2007**
(21) Application number: 05820149.2
(22) Date of filing: 22.12.2005
(51) Int. Cl.: A61B 1/00, G01N 33/50, A61B 19/00, G01N 33/68

(54) **FILTH FOR EVALUATING WASHING PERFORMANCE OF MEDICAL INSTRUMENT AND SOILING METHOD FOR EVALUATING WASHING PERFORMANCE**

(30) Priority: 27.12.2004 JP 2004378014; 27.12.2004 JP 2004378015; 17.01.2005 JP 2005009475
(71) Applicant: Olympus Corporation, Tokyo 151-0072 (JP)
(72) Inventor: NAGAI, Y. Olympus Medical Systems Corp., Tokyo 151-0072 (JP); KANAMORI, Yosuke Olympus Medical Systems Corp., Tokyo 151-0072 (JP); SATO, Sawako Olympus Medical Systems Corp., Tokyo 151-0072 (JP); NAKAO, Maiko Olympus Medical Systems Corp., Tokyo 151-0072 (JP); OBI, Kaori Olympus Medical Systems Corp., Tokyo 151-0072 (JP)
(74) Representative: von Hellfeld, Axel
(86) International application number: PCT/JP2005/023658
(87) International publication number: WO 2006/070699

(57) **Abstract**

With respect to evaluation of the efficacy of cleaning of a medical apparatus, the present invention provides a cleaning evaluation filth for a medical apparatus, the filth simulating a body fluid in a human body cavity and exhibiting good reproducibility of evaluation of the efficacy of cleaning. With respect to the cleaning evaluation filth of the present invention, proteins and lipids having concentrations for forming at least bile and serum, which are primary components for constituting a human digestive juice, are produced by dispensing reagents of proteins and lipids.

## Description

### Technical Field

The present invention relates to cleaning evaluation filth to be used for evaluating the efficacy of cleaning of a medical apparatus and a method for contaminating a medical apparatus with filth in order to evaluate the efficiency of a cleaning treatment of the medical apparatus with respect to a reusable medical apparatus.

### Background Art

In recent years, endoscopes have been frequently used because the endoscope can be inserted into a body cavity and, thereby, an inspection and a therapy of an organ can be performed. The endoscope must always be kept sanitary by being subjected to a cleaning/disinfecting treatment after each use in inspection or therapy.

With respect to various medical apparatuses including endoscopes, the efficacy of cleaning of a medical apparatus has been evaluated. That is, when a medical apparatus with filth adhered is subjected to a cleaning/disinfecting treatment, the efficacy of cleaning is evaluated on the basis of the presence of the filth remaining or the amount, the site, and the like of the filth remaining in the medical apparatus. Methods for checking the effect of the cleaning/disinfecting treatment of medical apparatuses have been proposed in, for example, US Patent No. 6,428,746.

On the other hand, the endoscope, which is a medical apparatus to be subjected to the cleaning/disinfecting treatment, is provided with channels, for example, a forceps channel for inserting various forceps to perform therapies and treatments of affected areas and a suction channel for suctioning water supplied forward for irrigating the affected area in a body cavity, cleaning water of an observation window at a distal end of an insertion portion of the endoscope, and the like.

Many kinds of filth have been applied in order to evaluate the efficacy of cleaning of medical apparatuses having the channels. The filth to be injected in the channel of a medical apparatus in order to evaluate the efficacy of cleaning is selected regardless of the properties of filth, e.g., the viscosity, while priority is given to bringing about the state in which the filth is adhered to the surface of the channel or the state in which a predetermined amount of filth remains in the channel.

On the other hand, it is known that if a filth having a high viscosity is adhered to a medical apparatus having a channel, a liquid pool of solid results in the channel.

The high viscosity of the filth to be used for evaluating the efficacy of cleaning of a medical apparatus having a channel causes a liquid pool of a filth generated in the channel of the medical apparatus. If the liquid pool is generated in the channel, the drying of the filth is not facilitated, because air do not pass through the channel. If the inside of the channel, in which the liquid pool of filth is present, is dried forcedly, the filth may be dried and solidified so as to cause clogging of the channel. Furthermore, since the state of contamination in the inside of the channel due to the filth is not stable, it is difficult to evaluate the efficacy of cleaning properly.

For example, in the case where a liquid pool is present in the inside of the channel due to the viscosity of the filth, even when an appropriate drying time is elapsed after the channel of the endoscope is contaminated by using a cleaning evaluation filth having a high viscosity, the drying of the channel is not facilitated. If the drying time is too extended, the liquid pool is dried and may cause clogging of the channel, so that it becomes difficult to stabilize the state of contamination in the inside the channel. If the state of contamination is not stabilized, there is a problem in that the efficacy of cleaning may not be evaluated properly.

As described above, with respect to various medical apparatuses to be used in the medical field, a cleaning treatment after use is important for preventing an infectious accident due to reuse of a medical apparatus after the use in diagnosis or therapy. Consequently, various cleaning apparatuses for performing cleaning treatments of medical apparatuses have been developed and put into practical use.

On the other hand, in the development and manufacture of a medical apparatus, for example, the efficacy of cleaning of a medical apparatus must be evaluated from a filth adhered to the medical apparatus when the medical apparatus is subjected to a cleaning treatment with a cleaning apparatus. The efficacy of cleaning of the medical apparatus is evaluated on the basis of the site, at which a filth remains, of the medical apparatus, the amount of filth remaining, and the like after the medical apparatus is actually used for a human body and an adhered filth is cleaned with a cleaning apparatus.

Consequently, since the evaluation of the efficacy of cleaning of the medical apparatus can be performed under limited conditions only, the evaluation takes much time, and the evaluation cannot be performed speedily. Furthermore, similar problems occur in the case where the cleaning is performed manually.

On the other hand, with respect to sanitary earthenware and enameled products, filths to be used for evaluating the efficacy of cleaning of the sanitary earthenware and the enameled products are proposed in Japanese Unexamined Patent Application Publication No. 2000-352101 and Japanese Unexamined Patent Application Publication No. 2001-104192. In Japanese Unexamined Patent Application Publication No. 2000-352101, an artificial filth in which a colorant is added to an aliphatic acid that is a primary component constituting human excreta is proposed in order to evaluate the manner of adhesion of a filth remaining or the manner of flushing when the filth adhered to the sanitary earthenware is flushed. Moreover, in Japanese Unexamined Patent Application Publication No. 2001-104192, an artificial filth formed from an aliphatic acid that is a major component of, for example, scales of a bathtub and filth by handling or oil stain of a kitchen door or a panel is proposed in order to evaluate the manner of adhesion of a filth remaining when the filth adhered to the enameled product is cleaned with water.

As is proposed in the above-described Japanese Unexamined Patent Application Publication No. 2000-352101 and Japanese Unexamined Patent Application Publication No. 2001-104192, the artificial filths for evaluating the efficacy of cleaning of the sanitary earthenware and the enameled products are proposed. However, an artificial filth having components and a viscosity close to, for example, those of human body fluids for evaluating the efficacy of cleaning of a reusable medical apparatus, e.g., an endoscope, has not been proposed. Consequently, realization of a cleaning evaluation filth for a medical apparatus has been desired, the filth being suitable for performing the evaluation of the efficacy of cleaning of a medical apparatus speedily in a short time and repeatedly under the same condition.

In recent years, various medical apparatuses including reusable endoscopes are surely cleaned and disinfected or sterilized after use in medical practice from the view point of prevention of infection. Various methods are used as the method for cleaning, disinfecting, and sterilizing medical apparatuses. The efficacy of cleaning of the medical apparatuses cleaned by various cleaning methods is evaluated on the basis of the amount of filth remaining in the medical apparatuses after the cleaning. Therefore, it is very important that the filth to be used for evaluating the efficacy of cleaning of the medical apparatus can be recovered after the cleaning and the composition of the filth is suitable for checking the state of filth remaining in the medical apparatus after the cleaning.

An artificial filth to be used for evaluating the efficacy of cleaning has been proposed in, for example, Japanese Unexamined Patent Application Publication No. 2000-352101. The artificial filth proposed in Japanese Unexamined Patent Application Publication No. 2000-352101 is used for evaluating the manner of adhesion of a filth remaining or the manner of flushing when the filth adhered to the sanitary earthenware is flushed.

Furthermore, an artificial filth to be used for evaluating the efficacy of cleaning medical apparatuses has been proposed in, for example, US Patent No. 6,447,990. Moreover, artificial filths produced primarily from various foods in combination are frequently used for evaluating the efficacy of cleaning of medical apparatuses.

The artificial filth proposed in Japanese Unexamined Patent Application Publication No. 2000-352101 is configured for the purpose of evaluating the efficacy of cleaning of the sanitary earthenware. With respect to the artificial filth, methods for using artificial filths, cleaning loads, e.g., ease of removal and resistance to removal in the cleaning, applicable cleaning methods, and the like are different between the reusable medical apparatus and the sanitary earthenware. The above-described artificial filth is unsuitable for the use in evaluation of the efficacy of cleaning of the medical apparatus.

The artificial filth proposed in US Patent 6,447,990 is used in evaluation of the efficacy of cleaning of reusable medical apparatuses. The artificial filth is configured while attention is given to human living body components which adhere during use of medical apparatuses and the concentrations of the components. However, no consideration is given to the cleaning loads, e.g., ease of removal and resistance to removal.

That is, the components of filths, which are living bodies, adhered to medical apparatuses are different depending on the sites of human body subjected to diagnosis and therapy. Furthermore, the sites, at which filths remain, of medical apparatuses are different depending on the shapes and the structures of the medical apparatuses. Consequently, evaluations must be performed with various cleaning methods and cleaning apparatuses, while the adhesion state of the filth, that is, the state of the cleaning load, in accordance with components of the filth or the site, at which filth remains, of medical apparatus are simulated.

With respect to the artificial filths produced from various foods in combination, the cleaning load is not taken into consideration as in the artificial filth of US Patent No. 6,447,990. Moreover, since the foods are perishable foods, the performance of the produced artificial filth varies depending on a climate, a source, and a manufacturer, there is a reproducibility problem in evaluation.

Accordingly, with respect to evaluation of the efficacy of cleaning of a medical apparatus, it is an object of the present invention to provide a cleaning evaluation filth for a medical apparatus, the filth simulating a body fluid in a human body cavity and exhibiting good reproducibility of evaluation of the efficacy of cleaning.

Furthermore, with respect to a filth for evaluating the efficacy of cleaning of a medical apparatus including a reusable endoscope, it is an object of the present invention to provide a cleaning evaluation filth for a medical apparatus with reproducibility in consideration of re-creation of the state of contamination similar to the clinical situation, in which the medical apparatus is actually used for a human body, by using the artificial filth and selection of a cleaning technique in accordance with the state of contamination.

Moreover, in consideration of the above-described circumstances, it is an object of the present invention to provide a contamination method for cleaning evaluation of a medical apparatus, wherein a liquid pool state of a filth inserted into a channel in order to evaluate the efficacy of cleaning of the channel of a medical apparatus is removed, a stable state of contamination is created and, thereby, proper evaluation of the efficacy of cleaning is made possible.

### Disclosure of Invention

### Means for Solving the Problem

A cleaning evaluation filth for a medical apparatus according to an aspect of the present invention is composed of an artificial filth prepared only from reagents configured to have components and concentrations simulating a human digestive juice.

A cleaning evaluation filth for a medical apparatus according to an aspect of the present invention is composed of an artificial filth which includes a solution containing a substance serving as an index for evaluating the efficacy of cleaning of the medical apparatus and an adhesion-promoting substance contained in the solution in order to adhere the solution to a cleaning evaluation site of the medical apparatus and which is produced by dispensing commercially available reagents.

A contamination method for cleaning evaluation of a medical apparatus according to an aspect of the present invention includes a filth injection step of injecting a filth into a channel of the medical apparatus and a contaminated layer formation step of forming a contaminated layer on a channel inner surface by supplying air into or suctioning air from the inside of the channel, into which the filth has been injected in the filth injection step.

### Brief Description of the Drawings

Fig. 1 is an explanatory diagram showing a first method of a filth injection step, according to a first embodiment.
Fig. 2 is an explanatory diagram showing a second method of the filth injection step, according to the first embodiment.
Fig. 3 is an explanatory diagram showing a first method of a filth layer formation step, according to the first embodiment.
Fig. 4 is an explanatory diagram showing a second method of the filth layer formation step, according to the first embodiment.
Fig. 5 is an explanatory diagram showing a third method of the filth layer formation step, according to the first embodiment.
Fig. 6A is a diagram showing the state of a filth in a channel by the filth injection step, according to the first embodiment.
Fig. 6B is a sectional view showing a contaminated layer formed on a channel inner surface by the filth layer formation step according to the first embodiment.
Fig. 7 is an explanatory diagram showing a first method of an endoscope channel filth injection step, according to a second embodiment.
Fig. 8 is an explanatory diagram showing a second method of the endoscope channel filth injection step, according to the second embodiment.
Fig. 9 is an explanatory diagram showing a third method of the endoscope channel filth injection step, according to the second embodiment.
Fig. 10 is an explanatory diagram showing a fourth method of the endoscope channel filth injection step, according to the second embodiment.
Fig. 11 is an explanatory diagram showing a first method of an endoscope channel filth layer formation step, according to the second embodiment.
Fig. 12 is an explanatory diagram showing a second method of the endoscope channel filth layer formation step, according to the second embodiment.
Fig. 13 is an explanatory diagram showing a third method of the endoscope channel filth layer formation step, according to the second embodiment.

### Best Mode for Carrying Out the Invention

A contamination method for cleaning evaluation of a medical apparatus according to embodiments of the present invention will be described below in detail with reference to the drawings.

### (FIRST EMBODIMENT)

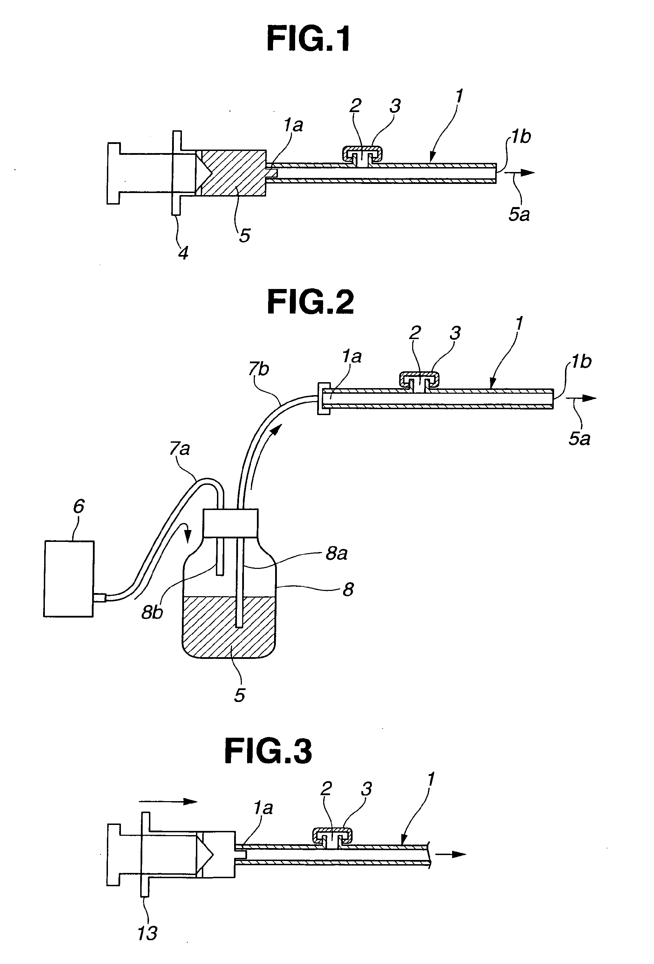

The contamination method for cleaning evaluation of a medical apparatus according to the first embodiment will be described below with reference to Fig. 1 to Fig. 6.

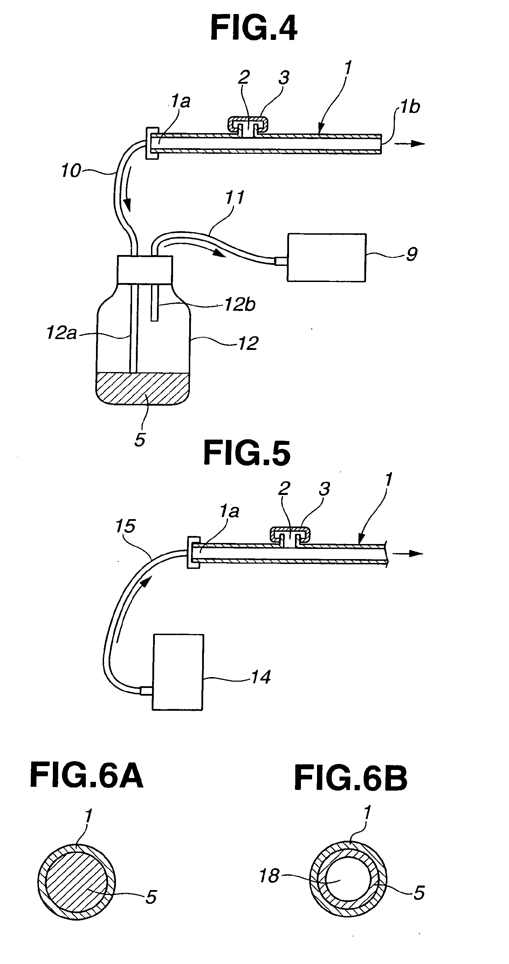

Fig. 1 is an explanatory diagram showing a first method of a filth injection step. Fig. 2 is an explanatory diagram showing a second method of the filth injection step. Fig. 3 is an explanatory diagram showing a first method of a filth layer formation step. Fig. 4 is an explanatory diagram showing a second method of the filth layer formation step. Fig. 5 is an explanatory diagram showing a third method of the filth layer formation step. Fig. 6A is a diagram showing the state of a filth in a channel by the filth injection step. Fig. 6B is a sectional view showing a contaminated layer formed on a channel inner surface by the filth layer formation step.

The contamination method for cleaning evaluation of a medical apparatus is composed of a filth injection step of injecting a filth into a channel and a contaminated layer formation step of forming a uniform contaminated layer on a channel inner surface without any liquid pool of the filth injected into the channel.

The first method of the filth injection step in the contamination method for cleaning evaluation of a medical apparatus will be described with reference to Fig. 1. The filth will be described later in detail.

A channel 1 of the medical apparatus basically has an opening 1a at one end (hereafter referred to as the one end opening 1a) and an opening 1 b at the other end (hereafter referred to as the other end opening 1b). In some cases, an opening 2 may be disposed at some midpoint in the channel 1 besides the openings 1 a and 1 b at the two ends of the channel 1 depending on the medical apparatus. The opening 2 is disposed in order to insert another medical apparatus into the inside of the channel 1 or connect an auxiliary apparatus of the medical apparatus.

In the case where the channel 1 has the opening 2, the opening 2 is hermetically sealed with a stopper 3 prior to the filth injection. Besides the stopper 3, the opening 2 may be hermetically sealed with any component, e.g., a button, insofar as the component can hermetically seal the opening 2.

After the opening 2 of the channel 1 is hermetically sealed, a syringe 4 storing a filth 5 is fitted to the one end opening 1a of the channel 1. The filth 5 is gradually injected into the inside of the channel 1 with the syringe 4 fitted to the one end opening 1 a of the channel 1. The filth 5 is injected with the syringe 4 until the filth 5 is filled in the channel 1 and outflows from the other end opening 1 b as a filth 5a. When it is ascertained that the filth 5 injected with the syringe 4 outflows from the other end opening 1b of the channel 1, the injection of the filth 5 with the syringe 4 is terminated.

Next, the second method of the filth injection step in the contamination method for cleaning evaluation of a medical apparatus will be described below with reference to Fig. 2. The same portions as those in Fig. 1 are indicated by the same reference numerals as in Fig. 1 and detailed explanations thereof will not be provided.

In the second method of the filth injection step, a tube 7b connected to a first glass tube 8a disposed in a filth container 8 storing a filth 5 is fitted to the one end opening 1a of the channel 1. A distal end of the first glass tube 8a is immersed in the filth 5 in the filth container 8. A second glass tube 8b is disposed in the filth container 8, and is connected to a blower 6 through a tube 7a. The second glass tube 8b is not immersed in the filth in the filth container 8, and the air supplied from the blower 6 is fed into the inside of the filth container 8.

That is, the air supplied from the blower 6 is fed into the filth container 8 through the second glass tube 7b and, thereby, the internal air pressure of the filth container 8 is increased. The filth 5 is injected into the channel 1 through the first glass tube 8a and the tube 7b by the increase of the internal air pressure. When it is ascertained that the filth 5 is injected and filled in the channel 1 by using the blower 6 and the filth container 8, the operation of the blower 6 is stopped and the injection of the filth is terminated.

When the filth 5 is injected into the channel 1 by any one of the first and the second methods of the filth injection step, as shown in Fig. 6A, the filth 5 is filled all over the surface of the channel 1.

Next, the first method of the contaminated layer formation step in the contamination method for cleaning evaluation of a medical apparatus will be described below with reference to Fig. 3. The same portions as those in Fig. 1 are indicated by the same reference numerals as in Fig. 1 and detailed explanations thereof will not be provided.

A syringe 13 storing air is fitted to the one end opening 1a of the channel 1 filled with the filth 5. The air in the syringe 13 fitted to the one end opening 1a of the channel 1 is injected into the inside of the channel 1. The filth 5 filled in the channel 1 is pushed toward the other end opening 1b by the air injected from the syringe 13. When the filth 5 in the channel 1 is pushed out by the air injected from the syringe 13, a path, through which the air has passed, is formed in the filth 5 between the one end opening 1a and the other end opening 1b in the channel 1. That is, a uniform contaminated layer is formed on an inner surface of the channel 1 without generating any liquid pool of the filth 5 by injecting the air into the filth 5 filled in the channel 1 in such a way that the air is injected until the formation of the path, through which the air has passed, is ascertained by a sound.

Subsequently, the second method of the contaminated layer formation step in the contamination method for cleaning evaluation of a medical apparatus will be described with reference to Fig. 4. The same portions as those in Fig. 1 are indicated by the same reference numerals as in Fig. 1 and detailed explanations thereof will not be provided.

A tube 10 connected to a first glass tube 12a disposed in a recovery container 12 for recovering the filth 5 is fitted to the one end opening 1a of the channel 1 filled with the filth 5. A distal end of the first glass tube 12a is arranged at a position which is immersed in the filth 5 recovered and accumulated in the recovery container 12. A second glass tube 12b is disposed in the recovery container 12, and the second glass tube 12b is connected to an aspirator 9 through a tube 11. A distal end of the second glass tube 8b is arranged at a position which is not immersed in the filth 5 recovered into the recovery container 12. The aspirator 9 suctions air in the inside of the recovery container 12.

That is, the air in the recovery container 12 is suctioned through the second glass tube 12b by suction with the aspirator 9 and, thereby, the internal air pressure of the recovery container 12 is decreased. The filth 5 filled in the channel 1 is suctioned through the first glass tube 12a and the tube 10 by the decrease of the internal air pressure, and is recovered into the recovery container 12. When the filth 5 in the channel 1 is suctioned by the suction of the aspirator 9, a path penetrating the filth 5 between the one end opening 1a and the other end opening 1b in the channel 1 is formed. That is, a contaminated layer of the filth 5 is formed on an inner surface of the channel 1 by suctioning the filth 5 filled in the channel 1 in such a way that the path penetrating the filth 5 is formed.

Furthermore, the third method of the contaminated layer formation step in the contamination method for cleaning evaluation of a medical apparatus will be described with reference to Fig. 5. The same portions as those in Fig. 1 are indicated by the same reference numerals as in Fig. 1 and detailed explanations thereof will not be provided.

A tube 15 from a blower 14 is fitted to the one end opening 1a of the channel 1 filled with the filth 5. The air is supplied from the blower 14 to the inside of the channel 1 through the tube 15 fitted to the one end opening 1a of the channel 1. The filth 5 filled in the channel 1 is pushed toward the other end opening 1b by the air supplied from the blower 14, and a penetration path is formed in the filth 5 between the one end opening 1 a and the other end opening 1 b in the channel 1. That is, a contaminated layer of the filth 5 is formed on an inner surface of the channel 1 by supplying the air to the filth 5 filled in the channel 1 so as to form the path penetrating the filth 5.

That is, when any one of the first to the third methods of the contaminated layer formation step is used, as shown in Fig. 6B, an air path 18, through which the air has passed, is formed in the filth 5 in the channel 1. Consequently, the filth 5 becomes in the state of being spread across an inner surface of the channel 1, and a uniform contaminated layer is formed without generating any liquid pool.

The contaminated layer of the filth 5 spread across the inner surface of the channel 1 while having the internal air path 18 from the one end opening 1 a to the other end opening 1b of the channel 1 prepared by the above-described filth injection step and the contaminated layer formation step is dried for an appropriate drying time. The drying of the contaminated layer may be performed by using thermo-hygrostat. The filth 5 to be used for contaminating the inside of the channel 1 may be a viscous filth or a known filth.

### (SECOND EMBODIMENT)

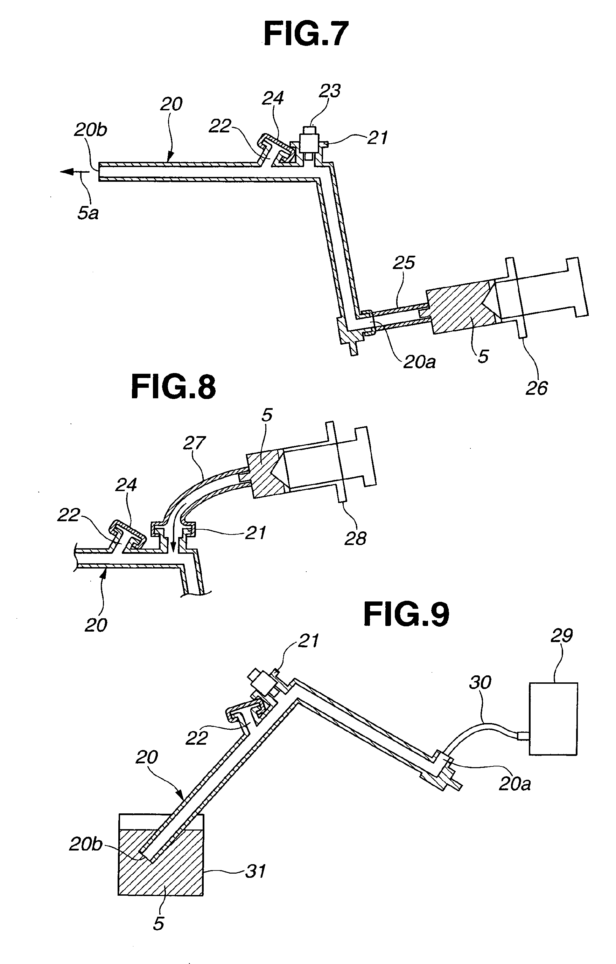

The second embodiment, in which the contamination method for cleaning evaluation of a medical apparatus is applied to a channel of an endoscope, will be described below with reference to Fig. 7 to Fig. 13.

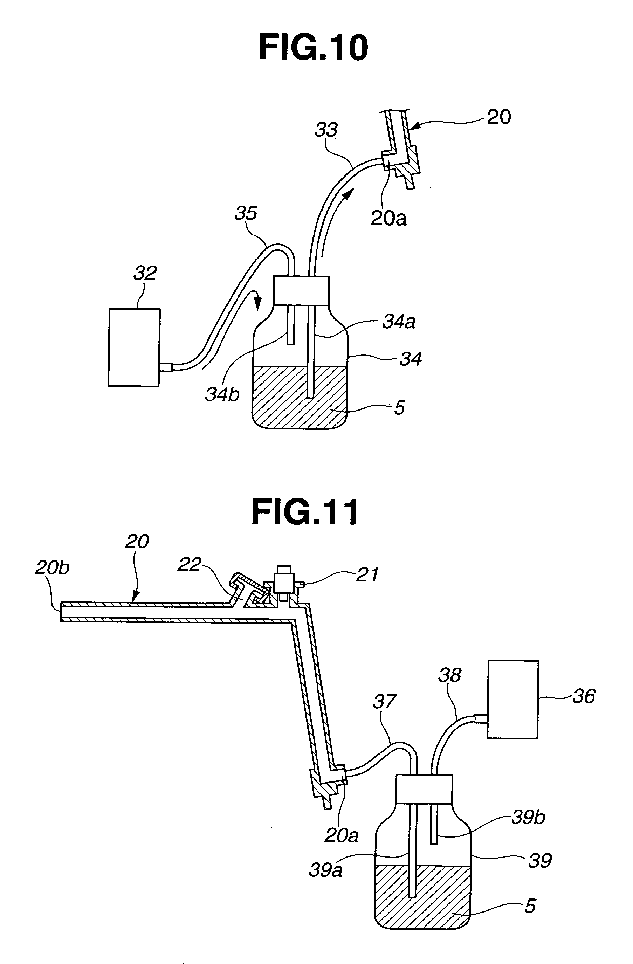

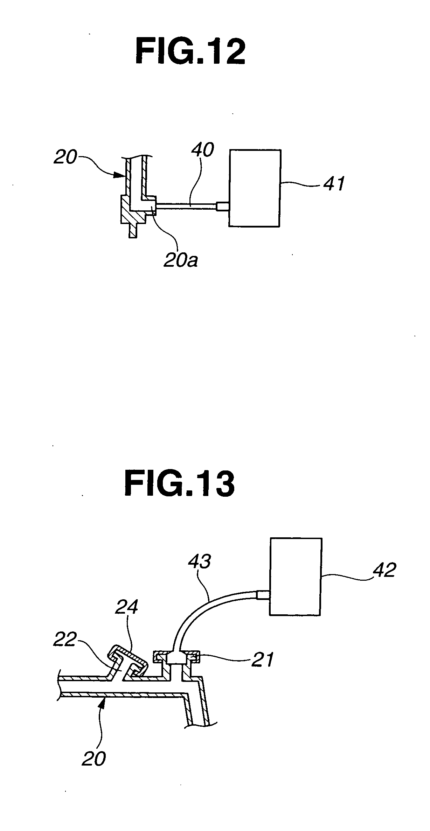

Fig. 7 is an explanatory diagram showing a first method of an endoscope channel filth injection step. Fig. 8 is an explanatory diagram showing a second method of the endoscope channel filth injection step. Fig. 9 is an explanatory diagram showing a third method of the endoscope channel filth injection step. Fig. 10 is an explanatory diagram showing a fourth method of the endoscope channel filth injection step. Fig. 11 is an explanatory diagram showing a first method of an endoscope channel filth layer formation step. Fig. 12 is an explanatory diagram showing a second method of the endoscope channel filth layer formation step. Fig. 13 is an explanatory diagram showing a third method of the endoscope channel filth layer formation step.

An endoscope channel 20 in the second embodiment will be described. The endoscope channel 20 is disposed in such a way as to communicate from a rear end opening 20a positioned on the connector side of the endoscope to a distal end opening 20b positioned at an insertion portion distal end through an operation portion and an insertion portion. The operation portion is provided with a cylinder portion 21 and a forceps opening 22, which communicate to the endoscope channel 20. The cylinder portion 21 is provided with an operation button 23 to perform operations of, for example, water supply toward the front from the distal end opening 20b of the endoscope channel 20 by a water supply pump fitted to the rear end opening 20a and suction through the distal end opening 20b of the endoscope channel 20 by a suction pump fitted to the rear end opening 20a. The forceps opening 22 is an opening disposed in order to insert forceps for various inspections or therapies into the endoscope channel 20.

That is, the forceps inserted from the forceps opening 22 into the endoscope channel 20 is protruded from the distal end opening 20b and, thereby, a living body of an affected area can be taken and a therapy can be performed. When a living body is taken by using forceps and an affected area is cleaned during therapy, the operator operates the operation button 23 so as to supply water from the distal end opening 20b.

The first method of the step of injecting a filth into the endoscope channel 20 will be described with reference to Fig. 7. Prior to the injection of the filth, the cylinder portion 21 is hermetically sealed with the operation button 23 or other sealing components. Furthermore, the forceps opening 22 is hermetically sealed with a forceps valve 24.

After the cylinder portion 21 and the forceps opening 22 are hermetically sealed, a syringe 26 storing the filth 5 is fitted to the rear end opening 20a of the endoscope channel 20 through a tube 25. The filth 5 is gradually injected into the inside of the endoscope channel 20 with the syringe 26 fitted to the rear end opening 20a of the endoscope channel 20. The filth 5 is injected with the syringe 26 until the filth 5 is filled in the endoscope channel 20 and outflows from the distal end opening 20b. When it is ascertained that the filth 5 injected with the syringe 26 outflows from the distal end opening 20b of the endoscope channel 20, the injection of the filth 5 with the syringe 26 is terminated.

Subsequently, the second method of the endoscope channel filth injection step will be described with reference to Fig. 8. The same portions as those in Fig. 7 are indicated by the same reference numerals as in Fig. 7 and detailed explanations thereof will not be provided.

In the second method of the filth injection step, the forceps opening 22 of the endoscope channel 20 is hermetically sealed, and a syringe 28 storing the filth 5 is fitted to the cylinder portion 21 through a tube 27. The filth 5 is injected into the endoscope channel 20 with the syringe 28 fitted to the cylinder portion 21. The filth 5 is injected with the syringe 26 until the filth 5 is filled in the endoscope channel 20 and outflows from the distal end opening 20b and the rear end opening 20a. When it is ascertained that the filth 5 injected with the syringe 26 outflows from the distal end opening 20b and the rear end opening 20a of the endoscope channel 20, the injection of the filth 5 with the syringe 26 is terminated.

The third method of the endoscope channel filth injection step will be described with reference to Fig. 9. The same portions as those in Fig. 7 are indicated by the same reference numerals as in Fig. 7 and detailed explanations thereof will not be provided.

In the third method of the filth injection step, the cylinder portion 21 and the forceps opening 22 of the endoscope channel 20 are hermetically sealed, and an aspirator 29 is fitted to the rear end opening 20a through a tube 30. The distal end opening 20b of the endoscope channel 20 is immersed in the filth 5 in a filth container 31. The filth 5 in the filth container 31 is suctioned through the distal end opening 20b of the endoscope channel 20 by the suction operation of the aspirator 29. When it is ascertained that the suctioned filth 5 outflows from the rear end opening 20a of the endoscope channel 20, the suction operation is terminated.

Further, the fourth method of the endoscope channel filth injection step will be described with reference to Fig. 10. The same portions as those in Fig. 7 are indicated by the same reference numerals as in Fig. 7 and detailed explanations thereof will not be provided.

In the fourth method of the filth injection step, the cylinder portion 21 and the forceps opening 22 of the endoscope channel 20 are hermetically sealed, and a tube 33 connected to a first glass tube 34a in a filth container 34 storing the filth 5 is fitted to the rear end opening 20a. A distal end of the first glass tube 34a is immersed in the filth 5 in the filth container 34. A second glass tube 34b connected to a blower 32 through a tube 35 is disposed in the filth container 34. A distal end of the second glass tube 34b is arranged at a position which is not in contact with the filth 5 in the filth container 34. When the blower 32 is operated and air is supplied to the inside of the filth container 34, the air pressure of the filth container 34 is increased. As a result, the filth 5 is injected into the endoscope channel 20 through the first glass tube 34a, the tube 33, and the rear end opening 20a. When it is ascertained that the filth 5 injected into the endoscope channel 20 outflows from the distal end opening 20b, the air supply operation is terminated.

The filth 5 is injected and filled in the endoscope channel 20 by any one of the above-described first to fourth methods.

Next, the first method of the endoscope channel contaminated layer formation step in the second embodiment will be described with reference to Fig. 11. The same portions as those in Fig. 7 are indicated by the same reference numerals as in Fig. 7 and detailed explanations thereof will not be provided.

The cylinder portion 21 and the forceps opening 22 are hermetically sealed, and a tube 37 connected to a first glass tube 39a disposed in a recovery container 39 is fitted to the rear end opening 20a of the endoscope channel 20. A distal end of the first glass tube 39a is arranged at a position which is immersed in the filth 5 recovered into the recovery container 39. A second glass tube 39b is disposed in the recovery container 39, and is connected to an aspirator 36 through a tube 38. A distal end of the second glass tube 39b is arranged at a position which is not in contact with the filth 5 recovered into the recovery container 39. When the aspirator 39 is operated to suction, the air pressure in the recovery container 39 is decreased. As a result, the filth 5 filled in the endoscope channel 20 connected to the first glass tube 39a through the tube 37 is suctioned. The suctioned filth 5 is recovered into the recovery container 39. When it is ascertained that the filth 5 in the endoscope channel 20 is suctioned and an air path is formed, the suction operation is terminated.

The second method of the endoscope channel contaminated layer formation step will be described below with reference to Fig. 12. The same portions as those in Fig. 7 are indicated by the same reference numerals as in Fig. 7 and detailed explanations thereof will not be provided.

The cylinder portion 21 and the forceps opening 22 are hermetically sealed, and a blower 41 is fitted to the rear end opening 20a of the endoscope channel 20 filled with the filth 5 through a tube 40. When the blower 41 is operated to supply air, the air is supplied to the endoscope channel 20 through the rear end opening 20a. The filth 5 filled in the endoscope channel 20 is pushed toward the distal end opening 20b by the supplied air. When it is ascertained that the filth 5 in the endoscope channel 20 is pushed out and an air path is formed, the air supply operation is terminated.

Subsequently, the third method of the endoscope channel contaminated layer formation step will be described below with reference to Fig. 13. The same portions as those in Fig. 7 are indicated by the same reference numerals as in Fig. 7 and detailed explanations thereof will not be provided.

The forceps opening 22 is hermetically sealed, and a blower 42 is fitted to the cylinder portion 21 of the endoscope channel 20 filled with the filth 5 through a tube 43. When the blower 42 is operated to supply air, the air is supplied to the endoscope channel 20 through the cylinder portion 21. The filth 5 filled in the endoscope channel 20 is pushed toward the distal end opening 20b and the rear end opening 20a by the supplied air. When it is ascertained that the filth 5 in the endoscope channel 20 is pushed out and an air path is formed, the air supply operation is terminated.

The air path, through which the air has passed, is formed in the filth 5 in the , endoscope channel 20 by using any one of the above-described first to third methods of the contaminated layer formation step. Consequently, the filth 5 becomes in the state of being spread across an inner surface of the endoscope channel 1, and a uniform contaminated layer is formed without generating any liquid pool.

In the state in which the air path is formed on the inner surface from the rear end opening 20a to the distal end opening 20b of the endoscope channel 20 and the filth 5 is spread across the inner surface of the endoscope channel 20 through the above-described filth injection step and the contaminated layer formation step, drying is performed for an appropriate drying time and, thereafter, the evaluation of the efficacy of cleaning is performed. The drying may be performed by using thermo-hygrostat. The filth 5 to be used for contaminating the inside of the endoscope channel 20 may be a viscous filth or a known filth.

The inventors of the present invention conducted comparative experiments in which quantitative measurement of protein in a channel of a medical apparatus including an endoscope was performed by the contamination method of the embodiment and a known contamination method. The results will be described with reference to Table 1 and Table 2. For the filth, BSA (bovine serum albumin) was used. For the measurement system, a micro BCA method (protein assay kit) was used. An operation, in which a filth is once injected and filled in a channel in the above-described filth injection step and excess filth in the channel is pushed out by suction or air supply so as to form an air path and a contaminated layer in the contaminated layer formation step, is referred to as aeration. That is, in Table 1, a term "without aeration" refers to the known contamination method and a term "with aeration" refers to a contamination method according to the embodiment.

**[Table 1]**

| | µg/suction channel | Log (logarithmic value) |
|---|---|---|
| | 157725 | 5.20 |
| Without aeration | 309900 | 5.49 |
| | 653474 | 5.82 |
| | 688275 | 5.84 |

**[Table 2]**

| | µg/suction channel | Log (logarithmic value) |
|---|---|---|
| | 246825 | 5.39 |
| With aeration | 214275 | 5.33 |
| | 222150 | 5.35 |
| | 219600 | 5.34 |

With respect to the evaluation of the efficacy of cleaning medical apparatuses, there are a case where the index is a germ and a case where the index is other substances, e.g., an organic material. In many cases, the evaluation of the efficacy of cleaning is performed on the basis of a subtracter of the index before the cleaning and the index after the cleaning, and the index is expressed as a logarithmic value Log. As shown in Table 1 and Table 2, the stability of the measurement values of the index protein with aeration is improved as compared with that without aeration. Furthermore, as a result of the contamination method according to the above-described embodiment, the logarithmic value Log of the index falls within the range of tolerance. Consequently, with respect to the evaluation of the efficacy of cleaning of a medical apparatus, in the situation in which the possibility of use of the evaluation based on the absolute value of the protein is discussed and expected, the stabilization of contamination by the aeration is indispensable. As a result, the contamination method according to the above-described embodiment is effective in the evaluation of the efficacy of cleaning of a medical apparatus.

Two examples of cleaning evaluation filths for a medical apparatus in the above-described first and second embodiments will be described below in detail. The cleaning evaluation filths for a medical apparatus, as described below, is used for evaluating the efficacy of cleaning of reusable medical apparatuses, e.g., endoscopes, which are inserted into body cavities and which are used for diagnoses and therapies of organs in the body cavities. For example, the endoscope serving as a medical apparatus is in a highest degree of contact with the digestive juice when being inserted into a body cavity.

The digestive juice is composed of various components. Among the components constituting the digestive juice, the components of the bile and the serum are contained as primary components at highest concentrations. The bile and the serum are primarily composed of proteins and lipids.

On the other hand, the proteins and the lipids constituting the bile and the serum are commercially available as reagents. An artificial digestive juice, that is, an artificial filth, can be produced by dispensing the reagents of the proteins and the lipids at the same concentrations as the concentrations of the bile and the serum contained in the digestive juice. That is, the same state as the clinical state of an actual use of a medical apparatus can be re-created by contaminating a cleaning evaluation site of the medical apparatus with the artificial filth serving as the artificial digestive juice.

Various experiments were conducted, in which commercially available reagents of the proteins and the lipids were dispensed and artificial filths were produced. As a result, it was made clear that the protein was prepared by dispensing 0.1 g/dl or more, and 8.0 g/dl or less of serum protein and 1.0 g/dl or more, and 4.0 g/dl or less of glycoprotein, the resulting protein was allowed to contain 0.5 g/dl or more, and 7.0 g/dl or less of phospholipid as the lipid, and thereby, the artificial filth was able to be produced.

Specifically, according to the experiments conducted by the inventors, when dispensation is performed by using 7.5 g/dl of serum albumin as the serum protein, 4.0 g/dl of mucin (for example, hog mucin) as the glycoprotein, and 5.0 g/dl of lecithin as the phospholipid, an artificial filth having concentrations composed of proteins and lipids, which are primary constituents of the bile and the serum of the digestive juice, can be produced. That is, an artificial filth which is composed of proteins and lipids and which has concentrations of the bile and the serum of digestive juice of the human body can be produced by blending predetermined weights or ratio of commercially available serum albumin, mucin (for example, hog mucin), and lecithin.

On the other hand, the efficacy of cleaning of a medical apparatus is evaluated by contaminating a cleaning evaluation site of the medical apparatus with a filth, performing cleaning with a cleaning apparatus or manually and, thereafter, recovering the filth remaining at the cleaning evaluation site of the medical apparatus. Examples of methods for recovering the remaining filth include an ultrasonic method, a wiping method, and a perfusion method by using a recovery solution. With respect to the evaluation of the efficacy of cleaning, the cleaning evaluation site of the medical apparatus may be visually observed and evaluated, or the remaining filth recovered from the cleaning evaluation site may be visually observed and evaluated. Alternatively, a method, in which the filth remaining at the cleaning evaluation site is recovered, quantitatively analyzed, and evaluated, may be used.

Quantitative methods, e.g., a Kjeldahl method, a Lowry method, and a BCA method, in which a protein serves as an index, are used as the quantitative analysis and evaluation method. Alternatively, the filth may be allowed to contain a germ, and quantification may be performed by using the germ as the index.

The BCA method is a quantitative method by using a protein, which is a component of the filth, as an index. In the BCA method, a medical apparatus contaminated with the filth is cleaned, the filth remaining at the cleaning evaluation site of the medical apparatus is recovered by using a recovery solution containing a surfactant, and the protein of the filth contained in the recovery solution is quantitatively measured. It is desirable that the concentration of the surfactant is less than or equal to the upper limit concentration set in the BCA method.

The BCA method can accurately quantify the protein without being affected by the properties of the filth nor the method for recovering the filth.

The micro BCA method may be used in place of the quantitative method based on the BCA method. In the micro BCA method, a sample of recovered artificial filth remaining on the medical apparatus used and cleaned is diluted appropriately with a recovery solution in such a way as to become within the protein measurement range of the micro BCA method, for example, 0.5 µg/ml or more, and 20.0 µg/ml or less, 1 ml of reaction reagent prepared in advance is added to 1 ml of sample of the remaining filth diluted with the recovery solution, and reaction is performed for 1 hour in an environment at 60°C. The sample after the reaction is cooled to room temperature and, thereafter, the absorbance is measured with the light of 562 nm. The amount of protein is calculated from the measurement result of the absorbance on the basis of the standard curve prepared separately. The protein measurement range of the micro BCA method is, for example 0.5 µg/ml or more, and 20.0 µg/ml or less. Therefore, the micro BCA method is most suitable for evaluation of cleaning of the medical apparatus after use, wherein with respect to the criteria of cleaning evaluation, it is required that the filth is significantly reduced by the cleaning.

In the case where the amount of protein of the filth remaining in the medical apparatus after cleaning is measured by using the BCA method or the micro BCA method, the evaluation of the efficacy of cleaning can be performed under the condition close to the clinical state by using the artificial filth produced from reagents in such a way as to simulate the same concentrations as the concentrations of the bile and the serum, which are highest concentration components among the components constituting the digestive juice of the human body.

The efficacy of cleaning of the medical apparatus contaminated with the above-described cleaning evaluation filth can be evaluated as a specific numerical value because the protein of the remaining filth can be quantified by using the BCA method or the micro BCA method.

Furthermore, since the above-described cleaning evaluation filth is produced by using commercially available reagents, excellent reproducibility of the concentration and the properties of the filth is exhibited, and with respect to the evaluation of the efficacy of cleaning of a medical apparatus, the quantitative evaluation under a constant condition becomes possible.

Subsequently, another example of the filth having a configuration different from the configuration of the above-described cleaning evaluation filth will be described below in detail.

With respect to the evaluation of the efficacy of cleaning of a medical apparatus, as described above, the evaluation is performed on the basis of the amount of filth remaining in the medical apparatus after the cleaning of the medical apparatus contaminated with the filth due to use in medical practice. In the method for evaluating the state of filth remaining after the cleaning of the medical apparatus, the filth remaining at the cleaning evaluation site of the medical apparatus is observed visually or with other observation means. Alternatively, the remaining filth is recovered from the cleaning evaluation site of the medical apparatus by using some type of means, and the recovered filth is observed visually, observed with other observation means, or subjected to quantitative analysis or the like.

With respect to observation means other than the visual observation, for example, dyeing with protein-bound coloring agent is performed in order to detect the remaining filth. In the quantitative analysis, the germ or the protein serving as an index is detected and analyzed. Examples of methods for detecting the germ include a method for measuring the number of germs by culture. Examples of methods for detecting the protein include a Lowry method and a BCA method.

In evaluation of the efficacy of cleaning, it is desirable that the efficacy of cleaning can be numerically evaluated on the basis of volumetric analysis in addition to the visual observation and evaluation. Among the medical apparatuses, the endoscope is inserted into a body cavity. Therefore, the endoscope is in a highest degree of contact with the digestive juice and adheres in the body cavity.

The cleaning evaluation filth for a medical apparatus, as described below, is an artificial filth composed of a solution containing a substance, e.g., a germ, serving as an index in the evaluation of the efficacy of cleaning, or a solution containing a protein which is a primary component of the digestive juice, and an adhesion substance which is added in order to ensure the adhesion of the solution containing the germ or protein to the cleaning evaluation site of the medical apparatus. The artificial filth is configured to include commercially available reagents, that is, serum albumin or the like as the protein and glycoprotein or the like as the adhesion substance. A hyaluronate may be used as glycoprotein.

That is, the artificial filth composed of the solution primarily containing the germ to be detected as the index in the method for measuring the number of germs by culture, the method being a detection method in quantitative analysis, or the protein to be detected as the index in the Lowry method and the BCA method and the adhesion substance which is added to the solution in order to adhere the solution to the medical apparatus is produced by using commercially available reagents. In order to evaluate and select a method for cleaning a medical apparatus and a cleaning technique, e.g., a cleaning apparatus, as described later, the adhesion substance is evaluated while the amount of addition (concentration) or the substance of the adhesion substance is changed and the adhesion to the cleaning efficacy evaluation site of the medical apparatus, that is, cleaning loads, e.g., ease of removal and resistance to removal is changed.

Next, the method for evaluating the efficacy of cleaning of a medical apparatus by using the above-described artificial filth will be described below. The above-described artificial filth is adhered to the cleaning efficacy evaluation site of an endoscope or other reusable medical apparatuses, test samples simulating the medical apparatuses, or the like and is stood for a predetermined time. The medical apparatus with the adhered artificial filth after being stood for the predetermined time is cleaned by a cleaning method, the efficacy of cleaning of which is to be evaluated. Examples of cleaning methods include a cleaning technique, e.g., manual cleaning which is cleaning by the hand of the surgeon, and cleaning with various cleaning apparatuses. With respect to the medical apparatus after the cleaning, the artificial filth remaining in the medical apparatus is evaluated by the above-described visual observation and, in addition, the remaining artificial filth is recovered and diluted with a predetermined solution, a protein serving as an index is subjected to volumetric analysis by, for example, the Lowry method or the BCA method, and the evaluation of the efficacy of cleaning is performed on the basis of the amount of the filth remaining after the cleaning.

In the evaluation of the efficacy of cleaning, the amount (concentration) or the substance of the adhesion substance to be added to the artificial filth is changed and, thereby, the adhesion of the filth to the medical apparatus is changed. That is, since the concentration of the filth adhered to the medical apparatus is varied depending on the diagnosis and therapy site of a human body and the adhesion state of the filth is changed depending on the site of the medical apparatus, a cleaning technique must be appropriately selected in accordance with the filth adhered and the site to be adhered. In order to select the cleaning technique, the contamination state heavier than or equivalent to a clinical state is re-created by using the artificial filth while the amount (concentration) or the substance of the adhesion substance is changed, the cleaning evaluation against the cleaning load is performed and, thereby, an optimum cleaning technique can be selected.

In the above-described artificial filth serving as a cleaning evaluation filth, the hyaluronate is used as the glycoprotein of the adhesion substrate. However, sodium hyaluronate may be used as the hyaluronate. It is preferable that the adhesion substance is determined in consideration of the concentration of the glycoprotein contained in the living body components which may adhere when the medical apparatus is actually used for therapy, a cleaning technique to be used for cleaning the medical apparatus, handling in the evaluation, and the like. According to the experiments conducted by the inventors in consideration of them, it is preferable that the concentration of the adhesion substance is about 10 mg/ml or less. In particular, when a difference between cleaning techniques is evaluated, a higher concentration is favorable. When the efficacy of cleaning of a lumer having such a small diameter as that of a flexible endoscope or the like is evaluated, optimization of the concentration is required in such a way that the artificial filth is surely adhered to the site at which the efficacy of cleaning is evaluated.

As described above, since the artificial filth is produced by using commercially available reagents, the efficacy of cleaning can be evaluated and the cleaning technique can be selected with excellent reproducibility.

According to the above-described embodiments, a contamination method for cleaning evaluation of a medical apparatus can be provided, wherein a liquid pool state of a filth inserted into a channel in order to evaluate the efficacy of cleaning of the channel of a medical apparatus is removed, a stable state of contamination is created and, thereby, proper evaluation of the efficacy of cleaning is made possible.

With respect to evaluation of the efficacy of cleaning of a medical apparatus, a cleaning evaluation filth for a medical apparatus can be provided, the filth simulating a body fluid in a human body cavity and exhibiting good reproducibility in evaluation of the efficacy of cleaning.

Furthermore, with respect to a filth for evaluating the efficacy of cleaning of a medical apparatus including a reusable endoscope, a cleaning evaluation filth for a medical apparatus with reproducibility can be provided in consideration of re-creation of the state of contamination similar to the clinical situation, in which the medical apparatus is actually used for a human body, by using the artificial filth and selection of a cleaning technique in accordance with the state of contamination.

## Claims

1. A cleaning evaluation filth for a medical apparatus, the filth comprising an artificial filth prepared only from reagents configured to have components and concentrations simulating a human digestive juice.

2. The cleaning evaluation filth for a medical apparatus according to Claim 1, wherein the artificial filth primarily simulates components and concentrations constituting bile and serum among the human digestive juice.

3. The cleaning evaluation filth for a medical apparatus according to Claim 2, wherein reagents of proteins and lipids constituting the bile and serum are used in the artificial filth.

4. The cleaning evaluation filth for a medical apparatus according to Claim 3, wherein reagents of serum protein and glycoprotein are used as the proteins and a reagent of phospholipid is used as the lipid in the artificial filth.

5. The cleaning evaluation filth for a medical apparatus according to Claim 4, wherein 0.1 g/dl or more, and 8.0 g/dl or less of serum protein and 1.0 g/dl or more, and 4.0 g/dl or less of glycoprotein serving as the proteins and 0.5 g/dl or more, and 7.0 g/dl or less of phospholipid serving as the lipid are dispensed and included in the artificial filth.

6. The cleaning evaluation filth for a medical apparatus according to Claim 5, wherein a reagent of serum albumin is used as the serum protein, a reagent of mucin is used as the glycoprotein, and a reagent of lecithin is used as the phospholipid in the artificial filth.

7. The cleaning evaluation filth for a medical apparatus according to Claim 6, wherein 7.5 g/dl serum albumin serving as the serum protein, 4.0 g/dl of mucin serving as the glycoprotein, and 5.0 g/dl of lecithin serving as the phospholipid are dispensed in the artificial filth.

8. A cleaning evaluation filth for a medical apparatus, the filth comprising an artificial filth which includes a solution containing a substance serving as an index for evaluating the efficacy of cleaning of the medical apparatus and an adhesion-promoting substance contained in the solution in order to adhere the solution to a cleaning evaluation site of the medical apparatus and which is produced by dispensing commercially available reagents.

9. The cleaning evaluation filth for a medical apparatus according to Claim 8, wherein the substance which is contained in the solution and which serves as the index for evaluating the efficacy of cleaning is a germ, a protein, e.g., serum albumin, or the like.

10. The cleaning evaluation filth for a medical apparatus according to Claim 8, wherein a glycoprotein or the like is used as the adhesion-promoting substance.

11. The cleaning evaluation filth for a medical apparatus according to Claim 10, wherein the glycoprotein serving as the adhesion-promoting substance includes a hyaluronate or sodium hyaluronate.

12. The cleaning evaluation filth for a medical apparatus according to Claim 11, wherein the concentration of hyaluronate or sodium hyaluronate, which is the glycoprotein serving as the adhesion-promoting substance, is 10 mg/ml or less.

13. A contamination method for cleaning evaluation of a medical apparatus, the method comprising:
a filth injection step of injecting filth into a channel of the medical apparatus; and
a contaminated layer formation step of forming a contaminated layer on a channel inner surface by supplying air into or suctioning air from the inside of the channel, into which the filth has been injected in the filth injection step.

14. The contamination method for cleaning evaluation of a medical apparatus according to Claim 13, wherein the filth is injected from one end of the channel of the medical apparatus and the filth is filled in the channel in such a way that the filth outflows from the other end in the filth injection step.

15. The contamination method for cleaning evaluation of a medical apparatus according to Claim 13, the method further comprising a clogging step of clogging openings other than a filth injection inlet at one end of the channel of the medical apparatus and a filth outflow outlet at the other end before the filth is injected.

16. The contamination method for cleaning evaluation of a medical apparatus according to Claim 13, wherein a uniform layer of the filth is formed on the channel inner surface by supplying air into or suctioning air from the channel, so as to form an air path penetrating the filth in the channel, in the contaminated layer formation step.
